(19) **Europäisches Patentamt**

**European Patent Office**

**Office européen des brevets**

(11) **EP 1 063 221 B1**

(12) **EUROPÄISCHE PATENTSCHRIFT**

(45) Veröffentlichungstag und Bekanntmachung des Hinweises auf die Patenterteilung:
**05.11.2003 Patentblatt 2003/45**

(51) Int Cl.$^7$: **C07C 47/058**, C07C 45/82, C07C 47/04

(21) Anmeldenummer: **00111802.5**

(22) Anmeldetag: **06.06.2000**

(54) **Verfahren zur Umsetzung einer ein Gemisch enthaltenden Lösung**

Process for the reaction of a solution containing a mixture

Procédé pour la réaction d'une solution contenant un mélange

(84) Benannte Vertragsstaaten:
**AT BE CH CY DE DK ES FI FR GB GR IE IT LI LU MC NL PT SE**

(30) Priorität: **07.06.1999 DE 19925870**

(43) Veröffentlichungstag der Anmeldung:
**27.12.2000 Patentblatt 2000/52**

(73) Patentinhaber: **BASF AKTIENGESELLSCHAFT**
**67056 Ludwigshafen (DE)**

(72) Erfinder:
• **Ströfer, Eckhard, Dr.**
**68163 Mannheim (DE)**

• **Scholl, Stephan, Dr.**
**67098 Bad Dürkheim (DE)**
• **Hasse, Hans, Prof. Dr.**
**67661 Kaiserslautern (DE)**

(74) Vertreter: **Isenbruck, Günter, Dr. et al**
**Isenbruck, Bösl, Hörschler, Wichmann, Huhn, Patentanwälte**
**Theodor-Heuss-Anlage 12**
**68165 Mannheim (DE)**

(56) Entgegenhaltungen:
**EP-A- 0 934 922        GB-A- 1 190 682**

**Beschreibung**

Die Erfindung betrifft ein Verfahren zur Umsetzung einer Lösung, die ein Gemisch von miteinander in einem chemischen Gleichgewicht stehenden chemischen Verbindungen enthält, mit wenigstens einer weiteren chemischen Verbindung.

Häufig liegen chemisch relevante Substanzen in gelöster Form als Gemisch von miteinander in einem chemischen Gleichgewicht stehenden chemischen Verbindungen (Komponenten) vor. Ein in der Praxis wichtiges Beispiel stellen wäßrige Lösungen von Formaldehyd dar. Formaldehyd ist ein wichtiger $C_1$-Baustein in der chemischen Industrie; die weltweite Produktion beläuft sich auf etwa 12 Millionen Jahrestonnen. Formaldehyd ist eine der reaktivsten organischen Komponenten und wird in wäßriger Lösung in Konzentrationen zwischen 20 und 55 % gehandelt und für eine Vielzahl chemischer Reaktionen eingesetzt. Eine wäßrige Formaldehydlösung enthält im Gleichgewicht ein Gemisch von Formaldehyd, Methylenglykol und Oligomeren aus mindestens zwei Methylenglykol-Einheiten, den Polyoximethylenglykolen. Alle diese Bestandteile wandeln sich in Abhängigkeit von z.B. pH-Wert und Temperatur mit endlicher Geschwindigkeit ineinander um. So degradieren z.B. die Polyoximethylenglykole bei erhöhter Temperatur zu Methylenglykol.

Chemische Reaktionen verlaufen in technischen Reaktoren im allgemeinen nicht mit einer Selektivität von 100 %. Die Selektivität zum gewünschten Hauptprodukt läßt sich aber durch geschickte Reaktionsführung steuern. Im Falle von Synthesen mit Formaldehyd liegt eine solche Steuerungsmöglichkeit in der Darbietungsform der wäßrigen Formaldehydlösung. So kann die Selektivität zum Hauptprodukt z.B. durch eine geeignete Einstellung der Konzentrationen von Formaldehyd, Methylenglykol und höheren Polyoximethylenglykolen optimiert werden.

Eine solche Einstellung der Konzentrationen ist bisher nur innerhalb enger Schranken durch eine geeignete Wahl des pH-Wertes, der Verdünnung und der Temperatur möglich.

Dasselbe gilt für eine Vielzahl von anderen Lösungen, die ein Gemisch von miteinander in einem chemischen Gleichgewicht stehenden chemischen Verbindungen enthalten.

Hiervon ausgehend stellt sich die Aufgabe, ein Verfahren zur Umsetzung solcher Lösungen anzugeben, das es ermöglicht, die Konzentration bestimmter Komponenten des Gemisches gezielt einzustellen und so die Selektivität der bei der Umsetzung ablaufenden chemischen Reaktionen zu steuern.

Ein solches Verfahren wird in der prioritätsälteren, nicht vorveröffentlichten DE-A-198 04 916 für die Umsetzung einer Formaldehydlösung, die Polyoximethylenglykole und gegebenenfalls monomeren Formaldehyd und/oder Methylenglykol enthält, mit Anilin in Gegenwart saurer Katalysatoren vorgeschlagen.

Die Aufgabe wird gelöst durch ein Verfahren zur Umsetzung einer Lösung, die ein Gemisch von mindestens zwei miteinander in einem chemischen Gleichgewicht stehenden chemischen Verbindungen enthält, mit wenigstens einer weiteren chemischen Verbindung, bei dem erfindungsgemäß folgende Schritte durchgeführt werden:

a) Auftrennung der Lösung durch ein Trennverfahren in wenigstens zwei Fraktionen, in denen unterschiedliche chemische Verbindungen des Gemisches im Vergleich zum chemischen Gleichgewicht angereichert sind; und

b) Umsetzung einer Fraktion mit der mindestens einen weiteren chemischen Verbindung vor vollständiger Wiedereinstellung des chemischen Gleichgewichts.

wobei die Lösung eine wässrige Formaldehydlösung, eine Lösung von Alkylthiolen in Schwefel oder eine Lösung von Oligomergemischen ist.

Durch den Einsatz eines geeigneten Trennverfahrens können die Konzentrationen bestimmter Komponenten des Gemisches gezielt in einem sehr weiten Bereich eingestellt werden. Die Konzentrationen der betreffenden Komponenten sind dabei unabhängig von Parametern, die den Reaktionsverlauf in unerwünschter Weise beeinflussen können, wie z.B. pH-Wert, Verdünnung oder Temperatur der Lösung.

Um sicherzustellen, daß sich die gewünschten angereicherten Komponenten vor der Umsetzung nicht wieder in die übrigen mit diesen im Gleichgewicht stehenden Komponenten umgewandelt haben, ist es hierbei sinnvoll, die Zeitdauer zwischen dem Schritt der Auftrennung der Lösung und dem Schritt der Umsetzung kürzer zu wählen als die Halbwertszeit des geschwindigkeitsbestimmenden Schrittes für die Einstellung des Gleichgewichts zwischen den Komponenten.

Vorteilhaft enthält das Trennverfahren mindestens einen Schritt, bei dem die Lösung zumindest teilweise verdampft wird. An die Verdampfung kann sich vorteilhaft eine wenigstens teilweise Kondensation anschließen.

Insbesondere eignet sich das erfindungsgemäße Verfahren, wenn die Lösung eine Lösung einer Oligomere bildenden Substanz in einem geeigneten Lösungsmittel ist.

Besonders vorteilhaft läßt sich das erfindungsgemäße Verfahren einsetzen, wenn die Lösung eine wäßrige Formaldehydlösung ist, die Formaldehyd, Methylen-glykol sowie Polyoximethylenglykole mit mindestens zwei Methylenglykol-Ein-heiten enthält.

Die nicht zur Umsetzung geführten Fraktionen können nach einer geeigneten Weiterbehandlung erneut in das Verfahren zurückgeführt werden. Besonders vorteilhaft ist es, wenn für mindestens eine der nicht zur Umsetzung geführten Fraktionen das Gleichgewicht durch geeignete Maßnahmen, z.B. einen Verweilzeitapparat, wenigstens teilweise wieder eingestellt wird und diese Fraktion dann erneut dem Trennverfahren zugeführt wird. Im einfachsten Falle handelt es sich um einen Verweilzeitbehälter als Verweilzeitapparat, in dem die Fraktion für eine gewisse Zeit belassen wird.

Um gleichmäßige und günstige Reaktionsbedingungen sicherzustellen, kann es sinnvoll sein, daß der dem Verweilzeitapparat zugeführten Fraktion Lösungsmittel entzogen wird. Dies kann geschehen, bevor oder nachdem diese Fraktion den Verweilzeitapparat durchläuft oder während sie sich in dem Verweilzeitapparat befindet.

Für den Schritt der Auftrennung in die unterschiedlichen Fraktionen können verschiedene Trennverfahren eingesetzt werden. Besondere Vorteile ergeben sich, wenn für die Auftrennung ein thermisches Trennverfahren in einem Filmverdampfer eingesetzt wird. Durch eine Vielzahl von Steuerungsmöglichkeiten, verschiedene mögliche Betriebsweisen und durch besondere Konstruktionsmerkmale erlaubt ein Filmverdampfer eine besonders gezielte Einstellung der Konzentrationen der Komponenten in den einzelnen Fraktionen. Des weiteren kann ein Filmverdampfer so ausgestaltet werden, daß ein Up- oder Downscaling (Maßstabsveränderung) problemlos möglich wird. So wird das erfindungsgemäße Verfahren maßstabsunabhängig in einer Vielzahl von Anwendungsfällen einsetzbar.

Besondere Ausführungsformen der vorliegenden Erfindung werden im folgenden anhand der beigefügten Zeichnungen erläutert. Es zeigt:

Fig. 1 einen schematischen Ablaufplan eines erfindungsgemäßen Verfahrens zur Umsetzung einer Lösung, die ein Gemisch von miteinander in einem chemischen Gleichgewicht stehenden chemischen Verbindungen enthält,

Fig. 2 die schematische Darstellung einer möglichen Ausführung eines Filmverdampfers, sowie

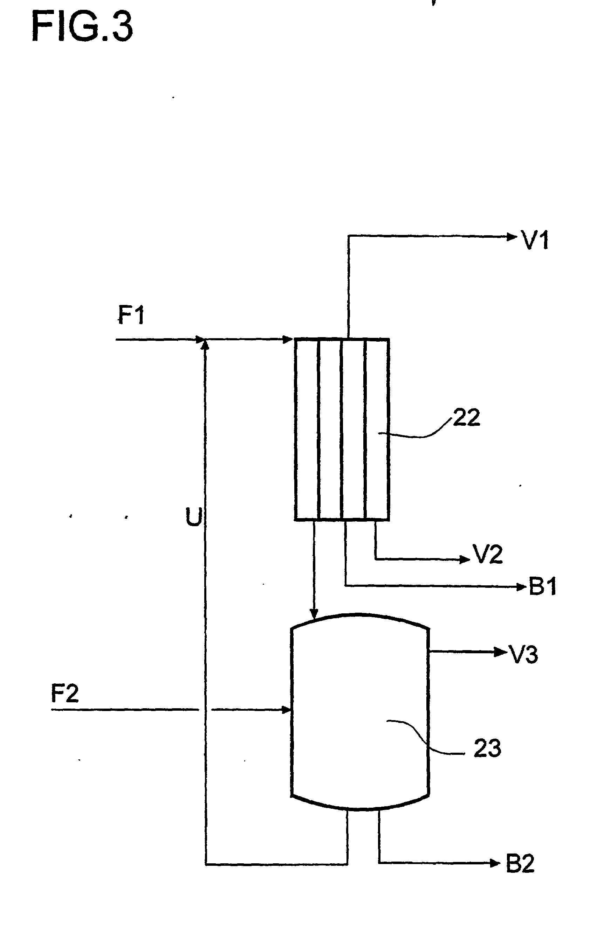

Fig. 3 eine Übersicht über verschiedene Betriebsweisen eines Filmverdampfers.

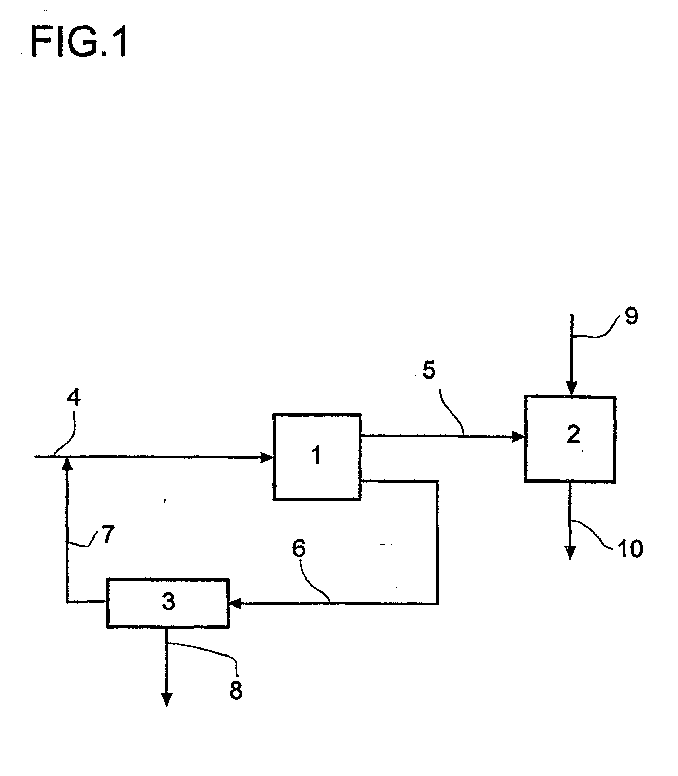

In der Fig. 1 ist schematisch der Ablauf eines erfindungsgemäßen Verfahrens beispielsweise zur Umsetzung einer wäßrigen Formaldehydlösung gezeigt. Über eine Zuleitung wird eine Formaldehyd enthaltende Rohlösung 4, z. B. handelsübliche 20 - 55 %ige Formaldehydlösung, zugeführt. Diese Lösung enthält mehrere, miteinander im chemischen Gleichgewicht stehende Komponenten: Formaldehyd (HCHO); Methylenglykol ($CH_2(OH)_2$), welches aus Formaldehyd durch Reaktion mit Wasser entsteht; sowie die Polyoximethylenglykole ($HO(CH_2O)_nH$, $n \geq 2$), welche durch Kondensation des Methylenglykols entstehen.

Die Rohlösung 4 wird einem Filmverdampfer 1 zugeführt. In diesem wird sie in eine erwünschte Fraktion 5 sowie eine Restfraktion 6 aufgetrennt. In der erwünschten Fraktion 5 sind z.B. Polyoximethylenglykole mit einem bestimmten Bereich von Kettenlängen n angereichert. Die erwünschte Fraktion wird sodann einem Hauptreaktorsystem 2 zugeführt, wo sie mit weiteren Edukten 9 zu Produkten 10 umgesetzt wird.

Die Zeitdauer zwischen dem Austreten der erwünschten Fraktion 5 aus dem Filmverdampfer und ihrer Umsetzung im Hauptreaktorsystem 2 wird dabei zweckmäßig so kurz gewählt, daß sich die Komponenten der Formaldehydlösung (Formaldehyd, Methylenglykol und Polyoximethylenglykole) in dieser Zeitdauer nicht in nennenswertem Maße ineinander umwandeln können. Insbesondere sollte die Zeitdauer kürzer sein als die Halbwertszeit des geschwindigkeitsbestimmenden Schrittes für die Einstellung des Gleichgewichts zwischen den Komponenten der Lösung unter den gegebenen Bedingungen. So ist z.B. bei einer Temperatur von 25°C die Zeitdauer zwischen dem Verlassen des Filmverdampfers und der Umsetzung möglichst kürzer als 300 bis 2500 s zu wählen, bei einer Temperatur von 80°C kürzer als 2 bis 10 s.

Die Restfraktion 6 wird einem Verweilzeitbehälter 3 zugeführt. In diesem verbleibt die Fraktion für eine Zeit, die ausreichend ist, das chemische Gleichgewicht zwischen Formaldehyd, Methylenglykol und den Polyoximethylenglykolen zumindest teilweise wiederherzustellen. In der Praxis beträgt diese Zeit zwischen 5 und 20 min bei Temperaturen zwischen 50 und 100°C. Gleichzeitig wird der im Verweilzeitbehälter 3 befindlichen Lösung mit geeigneten Mitteln (z.B. durch Destillation oder Extraktion) Wasser entzogen, um die Konzentration der gelösten Komponenten zu erhöhen. Dies ist durch die Wasserausschleusung 8 angedeutet. Der Entzug von Wasser kann auch vor dem Einleiten der unerwünschten Fraktion in den Verweilzeitbehälter 3 oder nach dem Austritt der Lösung aus dem Verweilzeitbehälter 3 erfolgen. Die so entstandene Lösung 7 wird danach der zugeführten Rohlösung 4 beigemischt und erneut in den Filmverdampfer 1 eingeleitet.

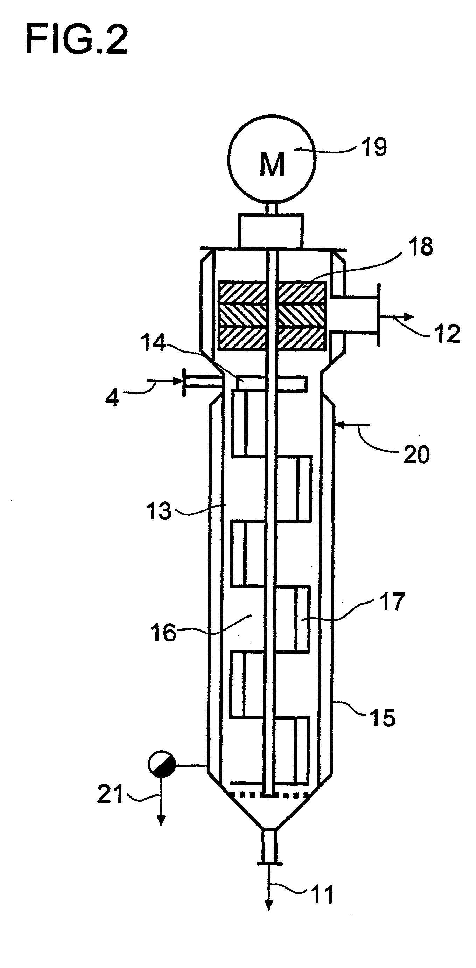

Ein für das beschriebene Verfahren besonders geeigneter Filmverdampfer ist in der Fig. 2 gezeigt. Es handelt sich hierbei um einen Dünnschichtverdampfer. Der Zulauf, bestehend aus Rohlösung 4 und gegebenenfalls Rückführstrom 7, wird zunächst einem Flüssigkeitsverteiler 14 zugeführt. Dieser verteilt die Rohlösung auf eine Verdampferfläche 13. Die Verdampferfläche 13 (Wärmeübertragungsfläche) ist üblicherweise zylindrisch geformt, kann jedoch auch zumindest teilweise konische Form aufweisen. Je nach Anwendungsfall kann sie z.B. aus Glas, Edelstahl, Email, Graphit oder einem geeigneten Kunststoff bestehen. Sie steht mit der Innenseite eines Heizmantels 15, der für eine Wärmezufuhr zur Verdampferfläche 13 sorgt, in thermischem Kontakt. Der Flüssigkeitsverteiler 14 trägt dazu bei, daß die Zulauflösung gleichmäßig auf den Umfang der Verdampferfläche 13 verteilt wird.

Rotierende Wischerblätter 17 verteilen die Lösung sodann weiter über die Verdampferfläche 13, sorgen für eine Aufrechterhaltung und Förderung eines Flüssigkeitsfilmes auf der Verdampferfläche 13 und tragen zur Intensivierung des flüssigkeitsseitigen Wärme- und Stofftransportes bei. Diese Wischerblätter 17 werden von einer Antriebsvorrichtung 19 angetrieben. Je nach Gestaltung und Positionierung des Wischorgans der Wischerblätter 17 kann dabei der Flüssigkeitsfilm eher dünn gehalten oder aufgestaut werden. Damit ist eine Veränderung der Verweilzeit bzw. der Verweilzeitverteilung der Lösung im Filmverdampfer möglich. Die typische Verweilzeit der Lösung im Filmverdampfer beträgt zwischen 1 s und 10 min, bevorzugt zwischen 2 s und 2 min.

Durch einen Heizmittelzulauf 20 wird ein Heizmittel, z.B. Wasserdampf, in den Heizmantel geführt. Dieses heizt die Verdampferfläche auf. Abgekühltes Heizmittel, z.B. kondensiertes Wasser im Falle von Wasserdampf als Heizmittel, wird über den Heizmittelablauf 21 abgeführt.

Durch die Wärmezufuhr zur Verdampferfläche 13 wird ein Teil der dem Filmverdampfer zugeführten Lösung verdampft, wodurch sich der nicht verdampfte Teil der Lösung in seiner Zusammensetzung verändert. Außerdem können in dem Verdampfer chemische Reaktionen der Komponenten des Gemisches ablaufen, die dem Verdampfungsprozeß überlagert sind und zu einer erhöhten Ausbeute an der gewünschten Komponente führen. Diese Reaktionen können auch vom pH-Wert oder/und von der Konzentration des Lösungsmittels abhängig sein. Diese Parameter können durch Zusatz von Lösungsmittel, Säure oder Lauge eingestellt werden.

Der entstandene Brüden (d.h. Dampf bzw. Gase) gelangt in einen Phasentrennraum 16 und von dort in einen Tropfenabscheider 18. Mit dem Brüden mitgerissene Flüssigkeitströpfchen werden hier aus der Gasphase entfernt und in die Flüssigkeit (Lösung) zurückgeführt. Das Konzentrat 11 wird auf geeignete Weise aus dem Phasentrennraum 16 ausgeleitet, während der Brüden 12 aus dem Tropfenabscheider 18 abgezogen wird. Der Brüden wird in einen nicht dargestellten Kondensator eingeleitet, wo er zumindest teilweise zu einem Kondensat kondensiert.

Wenn in den beschriebenen Filmverdampfer eine wäßrige Formaldehydlösung eingeleitet wird, reichem sich in der Flüssigkeit 11 die Polyoximethylenglykole an, während das Kondensat aus dem Brüden 12 arm an Polyoximethylenglykolen und reich an Formaldehyd und Methylenglykol ist. Auf diese Weise sind zwei Fraktionen, nämlich Konzentrat 11 und (Teil-)Kondensat aus dem Brüden 12 entstanden, in denen bestimmte Komponenten der ursprünglich zugeführten Rohlösung 4 selektiv angereichert sind.

Je nachdem, welche Reaktion im Hauptreaktorsystem 2 durchgeführt werden soll, kann in dem in der Fig. 1 gezeigten Verfahren die erwünschte Fraktion 5 mit dem Konzentrat 11 oder dem (Teil-)Kondensat identisch sein. Dementsprechend wird je nach Anwendung die jeweils andere Fraktion dem Verweilzeitbehälter 3 zugeführt.

Der Kondensator kann in einer besonderen Ausführungsform in den Verdampfungskörper integriert sein, wodurch sich eine kürzere Verweilzeit der verdampften Komponenten in der Dampfphase sowie eine kompaktere Bauweise ergeben.

Geeignete Betriebsbedingungen für den Filmverdampfer sind allgemein eine Temperatur zwischen 10°C und 230°C, bevorzugt zwischen 10°C und 150°C, bei einem Absolutdruck zwischen 0,5 mbar und 2 bar. Für die Auftrennung einer wäßrigen Formaldehydlösung sind Temperaturen zwischen 20°C und 100°C bei Normaldruck bevorzugt.

Neben der in Fig. 2 dargestellten Ausführung eines Filmverdampfers kann auch ein Apparat ohne mechanische Beeinflussung des Flüssigkeitsfilmes auf der Verdampfungsfläche eingesetzt werden. Die Wärmeübertragungsfläche dieser Fallfilm- oder Fallstromverdampfer kann dabei als Rohre oder Platten ausgebildet sein.

Abhängig von den konkreten Verfahrensanforderungen kann ein Filmverdampfer in verschiedenen Betriebsweisen eingesetzt werden. Die Fig. zeigt eine schematische Übersicht über die möglichen Betriebsweisen. Hierbei ist der eigentliche Filmverdampfer mit 20 und ein Brüdenabscheider (d.h. Phasentrennraum mit Tropfenabscheider) mit 21 bezeichnet. Sowohl der Filmverdampfer 20 als auch der Brüdenabscheider 21 können von der speziellen Bauform, wie sie in Fig. 2 dargestellt ist, abweichen und gegenüber der Fig. 2 weitere Zu- und Abläufe aufweisen. V1, V2 und V3 bezeichnen dampfförmige Ströme, alle anderen Ströme sind üblicherweise flüssig. Jeder der abgezogenen Ströme V1, V2, V3, B1 und B2 kann einzeln oder nach Vermischung (gegebenenfalls nach Kondensation) als erwünschte Fraktion der eigentlichen Umsetzung im Hauptreaktorsystem 2 zugeführt werden.

Der Filmverdampfer 22 kann bezüglich der austretenden, nicht verdampften Flüssigkeit im einmaligen Durchlauf oder in Kreislauffahrweise betrieben werden. Der Umlauf U ist technisch für einen Betrieb in Kreislauffahrweise notwendig. Er kann direkt oder über einen Verweilzeitbehälter erfolgen. Dieser Umlauf U ist nicht zu verwechseln mit der Rückführung 7 aus Fig. 1, welche von der technischen Betriebsweise des Filmverdampfers unabhängig erfolgen kann.

In nachstehender Tabelle sind die aktiven Ströme für die jeweils möglichen Betriebsweisen angegeben.

| | F1 | F2 | B1 | B2 | V1 | V2 | V3 | U |
|---|---|---|---|---|---|---|---|---|
| Einmaliger Durchlauf, Brüden und Flüssigkeit im Gegenstrom | X | | X | | X | | | |
| Einmaliger Durchlauf, Brüden und Flüssigkeit im Gleichstrom | X | | X | | | X | | |
| Kreislauffahrweise, Zulauf in Umlauf, Brüden und Flüssigkeit im Gleichstrom | X | | | X | | X | | X |
| Kreislauffahrweise, Zulauf in Umlauf, Brüden und Flüssigkeit im Gegenstrom | X | | | X | X | | | X |
| Kreislauffahrweise, Zulauf in Brüdenabscheider, Brüden und Flüssigkeit im Gleichstrom | | X | (X) | X | | X | (X) | X |
| Kreislauffahrweise, Zulauf in Brüdenabscheider, Brüden und Flüssigkeit im Gegenstrom | | X | | X | X | | (X) | X |

In einer Variante des erfindungsgemäßen Verfahrens bildet der Filmverdampfer selbst den Hauptreaktor 2 aus Fig. 1. In diesem Falle erfolgt die Umsetzung in situ im Filmverdampfer durch Zugabe wenigstens eines weiteren Eduktes 9 an geeigneter Stelle in den Filmverdampfer. Dies ermöglicht eine sehr kompakte Ausführung des gesamten Reaktorsystems und eine Minimierung der Transferzeit für die erwünschte Fraktion zwischen Auftrennung und Umsetzung.

Der Einsatz eines Filmverdampfers, insbesondere des in Fig. 2 dargestellten Dünnschichtverdampfers, für die Auftrennung der Rohlösung 4 in Fraktionen 5 und 6 bringt neben einem einfachen und zuverlässigen Betrieb eine Reihe weiterer Vorteile mit sich. So kann ein Filmverdampfer in verschiedenen Größen und für sehr verschiedene Massendurchsätze konstruiert werden. Ein Filmverdampfer kann sowohl in Verbindung mit einem Minireaktor mit einer Kapazität von weniger als 0,5 kg/h als auch in Verbindung mit einem Makroreaktor mit einer Kapazität von bis zu 30 t/h betrieben werden. Das erfindungsgemäße Verfahren ist damit extrem flexibel und kann für Spezialitäten im Pharmabereich bis hin zu Produkten großer Tonnage eingesetzt werden. Das Scale-up und Scale-down ist sowohl bezüglich der erreichbaren Stofftrennqualität als auch bezüglich der für eine gegebene Produktionsleistung erforderlichen Apparategröße problemlos möglich.

Für das beschriebene Verfahren ist eine Vielzahl von Anwendungsmöglichkeiten gegeben. Das Verfahren läßt sich überall dort vorteilhaft einsetzen, wo die Selektivität einer Reaktion von der Konzentration einer bestimmten Komponente der Rohlösung, verglichen mit den Konzentrationen der anderen Komponenten, abhängt.

Die Selektivität, mit der das Hauptprodukt gebildet wird, kann z.B. durch Parallelreaktionen gemindert werden, wobei die Geschwindigkeit der Haupt- und Nebenreaktion unterschiedliche Temperatur- und Konzentrationsabhängigkeiten aufweisen:

$$\text{HCHO, Methylenglykol} \quad + A \rightarrow \quad \text{Hauptprodukt}$$

$$+ A \rightarrow \quad \text{Nebenprodukt}$$

Ist z.B. A ein Amin, das methyliert werden soll, so ist es kinetisch vorteilhaft, wenn von Anbeginn an hohe Konzentrationen an freiem HCHO und/oder Methylenglykol vorliegen. Das Nebenprodukt ist in diesem Fall z.B. ein Kondensationsprodukt. Ist das Kondensationsprodukt das Hauptprodukt, so werden vorteilhaft höhere Polyoximethylenglykole statt HCHO und/oder Methylenglykol eingesetzt.

Die Selektivität kann auch gemindert werden durch Folgereaktionen des Hauptproduktes:

$$\text{HCHO, Methylenglykol} \quad + A \quad \rightarrow \quad \text{Hauptprodukt}$$

$$\text{HCHO, Methylenglykol} \quad + \text{Hauptprodukt} \quad \rightarrow \quad \text{Nebenprodukt}$$

Hier ist es wichtig, mit großen HCHO- bzw. Methylenglykol-Konzentrationen zu starten. Zwischenprodukte bei der Umsetzung von Aldehyden mit Formaldehyd können z.B. so vorteilhaft dargestellt werden.

Überlagern die chemische Reaktion und ein Mischvorgang, so kann es ebenfalls wichtig sein, die Konzentration an HCHO, Methylenglykol und höheren Polyoximethylenglykolen so einzustellen, daß die Selektivität zum Hauptprodukt optimiert wird.

Beispiele für Reaktionen, für die sich das erfindungsgemäße Verfahren einsetzen läßt, sind im Folgenden genannt:

Durch Umsetzung von Acetylen mit wäßriger Formaldehydlösung wird in einer Reppe-Reaktion Butindiol gewonnen, das zu Butandiol weiter hydriert wird. Durch Aldolisierungsreaktionen wird Formaldehyd mit sich selbst oder höheren Aldehyden zu mehrwertigen Alkoholen wie Zuckern, Pentaerythrit, Trimethylolpropan und Neopentylglykol umgesetzt. Aus wäßrigem Formaldehyd und CO kann Glykolsäure hergestellt werden. Chelatisierende Substanzen wie Glykolnitrile werden aus wäßrigen Lösungen von Formaldehyd hergestellt. In der Prins-Reaktion wird wäßriger Formaldehyd mit Olefinen zu alpha-Hydroxy-Methylverbindungen umgesetzt.

Wichtige Kondensationsreaktionen sind die Umsetzungen von wäßrigem Formaldehyd mit Aminen, wie z.B. Anilin oder Toluidin, zu Schiff'schen Basen. Durch Weiterreaktion entstehen Diphenylmethanderivate, wie z.B. Methandiphenyldiamin. Mit Hydroxylamin reagiert Formaldehyd zu Oximen. Wäßriger Formaldehyd und Diole bilden cyclische Ether, so entsteht aus Glykol und Formaldehyd Dioxolan.

Die Listung ist nicht vollständig. Lehrbücher der organischen Chemie und der technischen Chemie enthalten weitere Beispielreaktionen. Die Listung soll aber beispielhaft die industrielle Bedeutung des Formaldehyds als Synthesebaustein im gesamten Bereich der organischen Chemie verdeutlichen. Dies betrifft sowohl kleintonnagige Zwischenprodukte im Pharma- oder Pflanzenschutzbereich wie z.B. Oxime als auch großtonnagige Produkte wie Diphenylmethanderivate.

Das hier für wäßrige Formaldehydlösungen detailliert beschriebene Verfahren kann in ähnlicher Weise auch vorteilhaft für andere Lösungen eingesetzt werden, die ein Gemisch von miteinander in einem chemischen Gleichgewicht stehenden Komponenten enthalten. Dies gilt besonders für Lösungen, bei denen der gelöste Stoff eine Oligomere bildende Substanz ist. Solche Lösungen sind:

- Lösungen von Alkylthiolen in Schwefel, z.B. eine Methylmercaptan-(Methylthiol)-Lösung, die Dimethylsulfid, Dimethyldisulfid, Dimethyltrisulfid etc. enthält.
- Lösungen von Oligomergemischen aus Kondensationsreaktionen.

Bezugszeichenliste

| | |
|---|---|
| 1 | Filmverdampfer |
| 2 | Hauptreaktorsystem |
| 3 | Verweilzeitbehälter |
| 4 | Rohlösung |
| 5 | Erwünschte Fraktion |
| 6 | Unerwünschte Fraktion |
| 7 | Zurückgeführte Lösung |
| 8 | Ausschleusestrom |
| 9 | Edukte |
| 10 | Produkte |
| 11 | Konzentrat |
| 12 | Brüden |
| 13 | Verdampferfläche |
| 14 | Flüssigkeitsverteiler |
| 15 | Heizmantel |
| 16 | Phasentrennraum |
| 17 | Wischerblätter |
| 18 | Tropfenabscheider |
| 19 | Antrieb |
| 20 | Heizmittelzulauf |
| 21 | Heizmittelablauf |
| 22 | Filmverdampfer |
| 23 | Brüdenabscheider |

**Patentansprüche**

1. Verfahren zur Umsetzung einer Lösung, die ein Gemisch von mindestens zwei miteinander in einem chemischen Gleichgewicht stehenden chemischen Verbindungen enthält, mit wenigstens einer weiteren chemischen Verbindung (9), ausgenommen die Umsetzung einer Formaldehydlösung, die Polyoximethylenglykole und gegebenenfalls monomeren Formaldehyd und/oder Methylenglykol enthält, mit Anilin in Gegenwart saurer Katalysatoren, **dadurch gekennzeichnet, daß** folgende Schritte durchgeführt werden:

   (a) Auftrennung der Lösung durch ein Trennverfahren in wenigstens zwei Fraktionen (5, 6), in denen unterschiedliche chemische Verbindungen des Gemisches im Vergleich zum chemischen Gleichgewicht angereichert sind; und
   (b) Umsetzung einer Fraktion (5) mit der mindestens einen weiteren chemischen Verbindung (9) vor vollständiger Wiedereinstellung des chemischen Gleichgewichts,

   wobei die Lösung eine wässrige Formaldehydlösung, eine Lösung von Alkylthiolen in Schwefel oder eine Lösung von Oligomergemischen ist.

2. Verfahren nach Anspruch 1, **dadurch gekennzeichnet, dass** die Lösung eine wässrige Formaldehydlösung, enthaltend Formaldehyd, Methylenglykol und Polyoximethylenglykole, ist und mit wenigstens einer weiteren Verbindung aus der Gruppe Acetylen, Formaldehyd, CO, Olefine, Amine, Hydroxylamin und Diole umgesetzt wird.

3. Verfahren nach Anspruch 1 oder 2, **dadurch gekennzeichnet, daß** das Trennverfahren mindestens einen Schritt enthält, bei dem die Lösung zumindest teilweise verdampft wird.

4. Verfahren nach Anspruch 3, **dadurch gekennzeichnet, daß** sich an die Verdampfung eine zumindest teilweise Kondensation anschließt.

5. Verfahren nach einem der Ansprüche 1 bis 4, **dadurch gekennzeichnet, daß** für mindestens eine der nicht zur Umsetzung geführten Fraktionen das chemische Gleichgewicht wenigstens teilweise wiedereingestellt wird und diese Fraktion danach erneut dem Trennverfahren zugeführt wird.

6. Verfahren nach Anspruch 5, **dadurch gekennzeichnet, daß** die Wiedereinstellung des chemischen Gleichgewichtes in einem Verweilzeitapparat (3) erfolgt.

7. Verfahren nach Anspruch 6, **dadurch gekennzeichnet, daß** der dem Verweilzeitapparat (3) zugeführten Fraktion (6) Lösungsmittel entzogen wird.

8. Verfahren nach einem der Ansprüche 1 bis 7, **dadurch gekennzeichnet, daß** für die Auftrennung ein Filmverdampfer eingesetzt wird.

**Claims**

1. A method of reacting a solution comprising a mixture of at least two chemical compounds which are in chemical equilibrium with one another with at least one further chemical compound (9), with the exception of the reaction of a formaldehyde solution comprising polyoxymethylene glycols and possibly monomeric formaldehyde and/or methylene glycol with aniline in the presence of acid catalysts, which comprises the following steps:

   (a) fractionation of the solution by means of a separation method to give at least two fractions (5, 6) in which different chemical compounds of the mixture are present in increased concentrations compared to the chemical equilibrium; and
   (b) reaction of one fraction (5) with the further chemical compound (9) or compounds before the chemical equilibrium is fully reestablished,

   where the solution is an aqueous formaldehyde solution, a solution of alkyl thiols in sulfur or a solution of oligomer mixtures.

2. A method as claimed in claim 1, wherein the solution is an aqueous formaldehyde solution comprising formaldehyde, methylene glycol and polyoxymethylene glycols, and is reacted with at least one further compound from the group consisting of acetylene, formaldehyde, CO, olefins, amines, hydroxylamine and diols.

3. A method as claimed in claim 1 or 2, wherein the separation method comprises at least one step in which the solution is at least partly vaporized.

4. A method as claimed in claim 3, wherein the vaporization is followed by an at least partial condensation.

5. A method as claimed in any of claims 1 to 4, wherein chemical equilibrium is at least partially reestablished in at least one of the fractions not passed to the reaction and this fraction is then once again fed to the separation step.

6. A method as claimed in claim 5, wherein the reestablishment of the chemical equilibrium occurs in a residence time apparatus (3).

7. A method as claimed in claim 6, wherein the solvent is removed from the fraction (6) fed to the residence time apparatus (3).

8. A method as claimed in any of claims 1 to 7, wherein a film evaporator is used for the fractionation.

**Revendications**

1. Procédé pour la réaction d'une solution qui contient un mélange d'au moins deux composés chimiques se trouvant dans un équilibre chimique l'un avec l'autre, avec au moins un autre composé chimique (9), à l'exclusion de la réaction d'une solution de formaldéhyde, qui contient des polyoxyméthylèneglycols et éventuellement du formaldéhyde monomère et/ou du méthylèneglycol, avec de l'aniline en présence de catalyseurs acides, **caractérisé par le fait qu'**on effectue les étapes suivantes:

(a) décomposition de la solution par un procédé de séparation en au moins deux fractions (5, 6), dans lesquelles des composés chimiques différents du mélange sont enrichis par comparaison avec l'équilibre chimique;
et
(b) mise en réaction d'une fraction (5) avec le au moins un autre composé chimique (9) avant rétablissement complet de l'équilibre chimique,

tandis que la solution est une solution aqueuse de formaldéhyde, une solution d'alkylthiols dans du soufre ou une solution de mélanges d'oligomères.

2. Procédé selon la revendication 1, **caractérisé par le fait que** la solution est une solution aqueuse de formaldéhyde, contenant du formaldéhyde, du méthylèneglycol et des polyoxyméthylèneglycols, et est mise à réagir avec au moins un autre composé choisi du groupe de l'acétylène, du formaldéhyde, de CO, des oléfines, des amines, de l'hydroxylamine et des diols.

3. Procédé selon la revendication 1 ou 2, **caractérisé par le fait que** le procédé de séparation comprend au moins une étape dans laquelle la solution est vaporisée au moins partiellement.

4. Procédé selon la revendication 3, **caractérisé par le fait qu'**est adjointe à la vaporisation une condensation au moins partielle.

5. Procédé selon l'une des revendications 1 à 4, **caractérisé par le fait que** pour au moins l'une des fractions non soumises à la réaction on rétablit au moins partiellement l'équilibre chimique et on réintroduit ensuite cette fraction dans le procédé de séparation.

6. Procédé selon la revendication 5, **caractérisé par le fait que** le rétablissement de l'équilibre chimique s'effectue dans un appareil séparé à temps de séjour (3).

7. Procédé selon la revendication 6, **Caractérisé par le fait que** le solvant est extrait de la fraction (6) introduite dans l'appareil à temps de séjour (3).

8. Procédé selon l'une des revendications 1 à 7, **caractérisé par le fait que** pour la séparation on utilise un évaporateur pelliculaire.

# FIG.1

# FIG.2

# FIG.3